# EUROPEAN PATENT SPECIFICATION

(11) **EP 3 035 986 B1**
(45) Date of publication and mention of the grant of the patent: **29.04.2020**
(21) Application number: 14761499.4
(22) Date of filing: 22.08.2014
(51) Int. Cl.: A61M 5/168, A61M 5/315, A61M 5/00, G01D 5/165, A61M 25/00, A61B 6/00

(54) **VOLUME MONITORING DEVICE**
VOLUMENÜBERWACHUNGSVORRICHTUNG
DISPOSITIF DE SURVEILLANCE DE VOLUME

(30) Priority: 23.08.2013 US 201313975052
(43) Date of publication of application: 29.06.2016
(73) Proprietor: Osprey Medical, Inc., Minnetonka, Minnesota 55343 (US)
(72) Inventor: HOUFBURG, Rodney L., Prior Lake, MN 55372 (US); DOAN, Tuan Minh, Burnsville, MN 55337 (US)
(74) Representative: Vitillo, Giuseppe
(86) International application number: PCT/US2014/052319
(87) International publication number: WO 2015/027170

(56) References cited:
- EP-A1- 0 523 343
- WO-A1-96/11024
- WO-A2-2009/039203
- US-A- 4 006 736
- US-A- 5 662 612
- US-A1- 2003 233 069

## Description

### CROSS-REFERENCE TO RELATED APPLICATIONS

This application is being filed on 22 August 2014, as a PCT International patent application, and claims priority to U.S. Patent Application No. 13/975,052, filed August 23, 2013. The present application also claims the benefit under 35 U.S.C. § 120 and is a continuation-in-part of U.S. Patent Application No. 13/839,771, filed March 15, 2013, entitled "Devices and Methods for Modulating Medium Delivery," which claims the benefit under 35 U.S.C. § 119 to U.S. Provisional Application Ser. No. 61/694,137, filed August 28, 2012, entitled "Devices and Methods for Modulating Medium Delivery,".

### INTRODUCTION

This disclosure pertains to devices and methods used to control, transform or otherwise modulate the delivery of a substance, such as radiopaque contrast, to a delivery site and/or devices and methods that may be used to measure or otherwise make quantitative assessments of a medium delivered to a delivery site. More specifically, it is the intention of the following devices and methods to modulate and/or assess the delivery of media to a vessel, vascular bed, organ, and/or other corporeal structures so as optimize the delivery of media to the intended site, while reducing inadvertent or excessive introduction of the media to other vessels, vascular beds, organs, and/or other structures, including systemic introduction.

The terms medium (media), agent, substance, material, medicament, and the like, are used generically herein to describe a variety of fluidal materials that may include, at least in part, a substance used in the performance of a diagnostic, therapeutic or/and prophylactic medical procedure and such use is not intended to be limiting.

It's known from the patent application WP 96/11024 A1 an infusion pump for dispensing fluid from a syringe having a barrel and a plunger slidably inserted into the barrel. The infusion pump provides a housing having a bracket which supports the barrel. A syringe driver is movably attached to the housing such that the syringe driver abuts the plunger to slide the plunger into the barrel. A sensor is provided for sensing both the position of the plunger to the barrel and the capture of the plunger in the syringe driver. Control circuitry is electrically connected to the sensor for determining the capture and position of the plunger. The sensor comprises a potentiometer attached to the housing and electrically connected to the control circuitry. The sensor further comprises a wiper supported by the syringe driver. The wiper operatively contacts the potentiometer such that the control circuitry receives an output signal from the potentiometer to determine the position of the plunger and the capture of the syringe driver in the plunger.

The patent application US 5 662 612 A discloses a known computer-controlled injector of the type as a motor which advances and retracts a plunger located within a syringe housing toward and away from a nozzle located in the front of the syringe to inject fluid into or out of an animal subject. The injector tracks the plunger position during an injection, and compensates for plunger extenders found in partially pre-filled syringes by applying a stored offset value to the computed plunger position, to produce a corrected plunger position reflecting the actual position of the plunger and compensating for the presence of the extender. The offset value can be determined by querying the operator as to the capacity of the syringe and determining therefrom the appropriate offset value, or may be automatically computed by detecting physical indicia on the syringe or extender which indicate the length of the extender.

From the patent application US 4 006 736 A it's known an apparatus for injecting fluid, specifically contrast media, into the vascular system of a human being or other animal. The apparatus comprises a head portion and a control unit. The head portion includes the motor and drive components, and carries a removable syringe cartridge containing the fluid. Integral with the head portion is a pressure jacket for housing the syringe cartridge, and a piston plunger for forcing the fluid out of the syringe cartridge, through a catheter and into the vascular system. The control unit includes the mechanism for selecting delivery parameters, and circuitry for controlling the delivery of the contrast media into the vascular system. Also disclosed is a syringe cartridge designed to cooperate with the apparatus for injecting contrast media.

The patent application EP 0 523 343 A1 discloses a known syringe position detection and alarm system continuously determines the position of a syringe plunger and provides near end of travel and end travel alarms. Plunger movement is also monitored and if movement is less than a calculated amount, an alarm is provided. A variety of syringes of different sizes is usable. A potentiometer is mounted along the line of travel of the screw drive mechanism and a marker is mounted to extend from the screw mechanism to the potentiometer to force an internal contact in the potentiometer. The voltage provided by this internal contact is analyzed by the processor to derive absolute and relative position of the plunger. The end of travel position of each plunger is stored in processor memory and from the operator input rate of infusion, the characteristic curve of the potentiometer also stored in memory, the plunger displacement of the syringe, and the time of the near end of travel alarm, the voltage of the near end of travel position is calculated. The potentiometer is self-container and sealed.

It's known from the patent application WO 2009/039203 A2 a controller for closed loop control of infusion devices is described. A variety of infusion systems are described that use the closed loop control architecture. In some embodiments the closed loop control may be adapted to current commonly used infusion means such as a gravity feed intravenous system. Other embodiments describe infusion pump system that use biasing or drive mechanisms of springs, elastomers, rotary and linear motors.

Furthermore it's known from the patent application US 2003/233069 A1 an infusion pump, which is configured to be powered by either a disposable battery source or a rechargeable battery source. The infusion pump has a housing having a recess. A motor is positioned within the housing and is operably connected to an electrical contact disposed in the recess. The motor powers the pump. The recess receives one of a rechargeable battery unit having an electrical contact that contacts the recess electrical contact, and a disposable battery unit having an electrical contact that contacts the recess electrical contact.

### SUMMARY

This summary is provided to introduce a selection of concepts in a simplified form that are further described below in the Detailed Description. This summary is not intended to identify key features or essential features of the claimed subject matter, is not intended to describe each disclosed embodiment or every implementation of the claimed subject matter, and is not intended to be used as an aid in determining the scope of the claimed subject matter. Many other novel advantages, features, and relationships will become apparent as this description proceeds. The figures and the description that follow more particularly exemplify illustrative embodiments.

In one aspect, the technology relates to an apparatus including: a syringe housing; a plunger slidably received within the syringe housing; a plunger magnet secured to the plunger; a potentiometer housing fixed to the syringe housing; a potentiometer disposed within the potentiometer housing; and a wiper magnet slidably received within the potentiometer housing, wherein a movement of the plunger magnet causes a corresponding movement of the wiper magnet. In an embodiment, the apparatus includes a plurality of leads extending from the potentiometer. In another embodiment, the apparatus includes an interface for connecting the plurality of leads to a measuring device, and wherein the measuring device displays a total volume injected and emits a warning of a critical outcome. In yet another embodiment, the potentiometer housing is releasably fixed to the syringe housing. In still another embodiment, the apparatus includes means for releasably securing the potentiometer housing to the syringe housing.

In another embodiment of the above aspect, the means includes at least one of a clamp, a clasp, a hook and loop fastener, and a magnet. In another embodiment, the means for releasably securing the potentiometer is secured to the potentiometer housing. In another embodiment, the means for releasably securing is releasably secured to the potentiometer housing. In another aspect, the technology relates to an apparatus including: a syringe housing; a plunger slidably received within the syringe housing; a potentiometer secured to the syringe housing; a first magnet movably positionable relative to the potentiometer; and a second magnet, wherein the first magnet and the second magnet are adapted to move in tandem. In an embodiment, the apparatus includes a lead for sending an output signal from the potentiometer to an interface, wherein the interface displays a total volume injected and emits a warning of a critical outcome. In another embodiment, the output signal varies based on a position of the first magnet relative to the potentiometer. In yet another embodiment, the first magnet is disposed proximate the potentiometer. In still another embodiment, the second magnet is fixed to the plunger. In another embodiment, the second magnet includes a plurality of magnets

In another aspect, the technology relates to an apparatus including: a syringe housing; a potentiometer secured to the syringe housing; a first magnet movably positionable relative to the potentiometer; and a plunger slidably received within the syringe housing, wherein the first magnet is movable based at least in part on a movement of the plunger. In an embodiment, the apparatus a second magnet secured to the plunger and aligned with the first magnet. In another embodiment, the apparatus includes a third magnet secured to the plunger and aligned with the first magnet. In yet another embodiment, the first magnet is disposed within a magnetic field formed by at least one of the second magnet and the third magnet. In still another embodiment, the apparatus includes a potentiometer housing, wherein the potentiometer and the first magnet are disposed within the potentiometer housing. In another embodiment, the apparatus includes a lead for sending an output signal from the potentiometer to an interface, wherein the interface displays a total volume injected and emits a warning of a critical outcome.

### BRIEF DESCRIPTION OF THE DRAWINGS

There are shown in the drawings, embodiments which are presently preferred, it being understood, however, that the technology is not limited to the precise arrangements and instrumentalities shown.
FIG. 1A depicts an exemplary synchronized agent delivery with indirect modulation, adjacent a distal portion of a treatment system therefor.
FIG. 1B depicts an exemplary synchronized agent delivery with indirect modulation (top view), adjacent a proximal portion of such a treatment system.
FIG. 1C depicts an exemplary synchronized agent delivery with indirect modulation (side view), adjacent a proximal portion of such a treatment system.
FIG. 1D depicts, in side sectional view, the brake mechanism of the exemplary synchronized agent delivery arrangement of FIG. 1C.
Figures 2-6 show embodiments of the present invention. FIG. 2 depicts a perspective view of an embodiment of a monitoring syringe.
FIG. 3 depicts a partially exploded perspective view of the monitoring syringe of FIG. 2.
FIG. 4A depicts a partial enlarged perspective view of another embodiment of a monitoring syringe.
FIG. 4B depicts a partial enlarged perspective view of another embodiment of a monitoring syringe.
FIGS. 5A-5C depict a method of using a monitoring device.
FIG. 6 depicts a partially exploded perspective view of another embodiment of a monitoring syringe,

### DETAILED DESCRIPTION

There are numerous occasions in the diagnostic, prophylactic and treatment practice of medicine wherein an agent, medicant, or medium is preferably delivered to a specific site within the body, as opposed to a more general, systemic introduction. One such exemplary occasion is the delivery of contrast media to coronary vasculature in the diagnosis (i.e., angiography) and treatment (i.e., balloon angioplasty and stenting) of coronary vascular disease. The description, as well as the devices and methods described herein, may be used in modulating contrast media and/or monitoring the delivery to the coronary vasculature in prevention of toxic systemic effects of such an agent. One skilled in the art, however, would recognize that there are many other applications wherein the controlled delivery and/or quantitative assessment of a media to a specific vessel, structure, organ or site of the body may also benefit from the devices and methods disclosed herein. For simplicity, these devices and methods may be described as they relate to contrast media delivery modulation and/or measurement. As such, they may be used in the prevention of Contrast Induced Nephropathy; however, it is not intended, nor should it be construed, so as to limit the use to this sole purpose. Exemplary other uses may include the delivery, injection, modulation, or measurement of: cancer treatment agent to a tumor, thrombolytic to an occluded artery, occluding or sclerosing agent to a vascular malformation or diseased tissue; genetic agent to a muscular bed, neural cavity or organ, emulsion to the eye, bulking agent to musculature and/or sphincter, imaging agent to the lymphatic system, antibiotics to an infected tissue, supplements in the dialysis of the kidney, to name but a few.

### EXAMPLE - Prevention of Contrast Induced Nephropathy

Contrast Induced Nephropathy (CIN) is a form of kidney damage caused by the toxic effects of dyes (radiopaque contrast media) used, for example, by cardiologists to image the heart and its blood vessels during commonly performed heart procedures, such as angiography, angioplasty, and stenting. In general, the dye is toxic and is known to damage kidneys. Although most healthy patients tolerate some amount of the "toxicity," patients with poorly or non-functioning kidneys may suffer from rapidly declining health, poor quality of life, and significantly shortened life expectancy. Potential consequences of CIN include: irreversible damage to the kidneys, longer hospital stays, increased risk of heart disease, increased risk of long-term dialysis, and ultimately, a higher mortality risk. For patients who acquire CIN, their risk of dying remains higher than others without CIN, and this risk can continue up to five years after their procedure. CIN has a significant economic burden on the healthcare system and currently there is no treatment available to reverse damage to the kidneys or improper kidney performance, once a patient develops CIN.

To date, there have been attempts in reducing the toxic effects of contrast media on patients who undergo procedures involving dyes, especially those patients who are at high risk for developing CIN. Some of these efforts have been to: change the inherent toxicity (of a chemical or molecular nature) of the dyes, reduce the total amount of contrast agent injected (through injection management and/or dye concentration), and remove media through coronary vasculature isolation and blood/contrast agent collection systems, to name a few. These methods and devices used in the control of the toxic effects of contrast agents have had their inherent compromises in effectively delivering a contrast media specifically to a target site while minimizing the systemic effects. As an example, changing the composition of a dye and/or injection concentration may help reduce a contrast agent's inherent toxicity at the expense of the contrast agent's ability to perform its intended function (e.g., visualization of vasculature). Conversely, the ability to "collect" contrast agent laden blood "downstream" from the visualization site may ensure visualization, but requires the complexity of placement and operation of a collection system.

Other attempts to manage the amount of contrast agent delivered to a patient have employed automated, powered (versus manual, syringe-injected) contrast media injection systems. Close monitoring and control of the total quantity of contrast agent injected may have a positive impact in reducing the incidence of CIN. However, these injection systems are expensive (including capital equipment and disposables), cumbersome to use within a cath lab, and take additional time and expertise to set up and operate properly. Improper use could negate any benefits seen by better management of the quantity of the contrast agent delivered to a patient, and the additional time required to set up such a system may also add significant complexity to a procedure. The devices and methods described herein may measure or otherwise quantitatively assess the amount of medium injected or delivered to a delivery site using a relatively fast, simple, economical, and safe system.

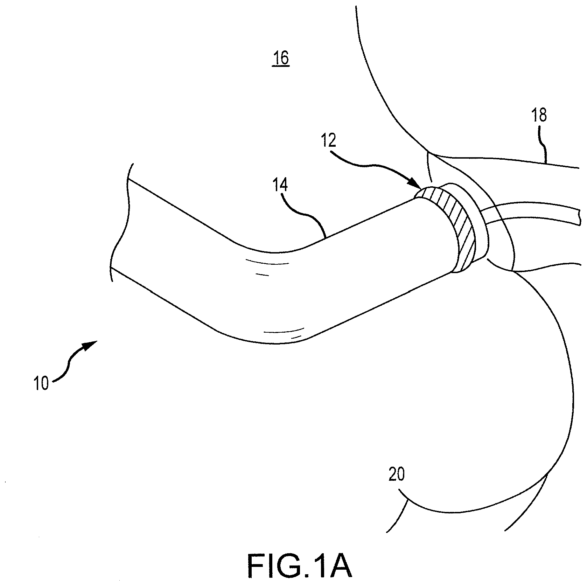

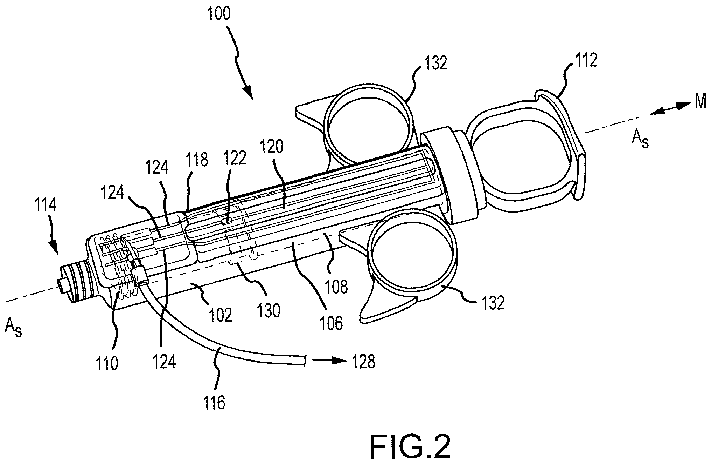

The measurement systems described herein may be employed as a system of quantitative assessment or in combination with a modulator. Additional systems are described in U.S. Patent Application No. 13/839,771. FIGS. 1A-1D depict embodiments where a modulator is constructed so as to measure the amount of an agent delivered from the system. Conversely, FIG. 2, for example, describes the use of a measurement system for the quantitative assessment of the volume of medium delivered and the inherent analysis of the total volume delivered versus some predetermined critical amount, such as the Gurm ratio, whether or not it is used with a modulator.

It should be understood that measurements may be performed prior to a medium being modulated, simultaneously with modulation, or after the modulation process, if desired. Further, it is also contemplated that the measurement devices and methods may be used with any of the modulation systems, such as described in U.S. Patent Application Ser. No. 13/839,771. Moreover, the embodiments described herein are exemplary in nature and should not be construed as limiting the various combinations possible.

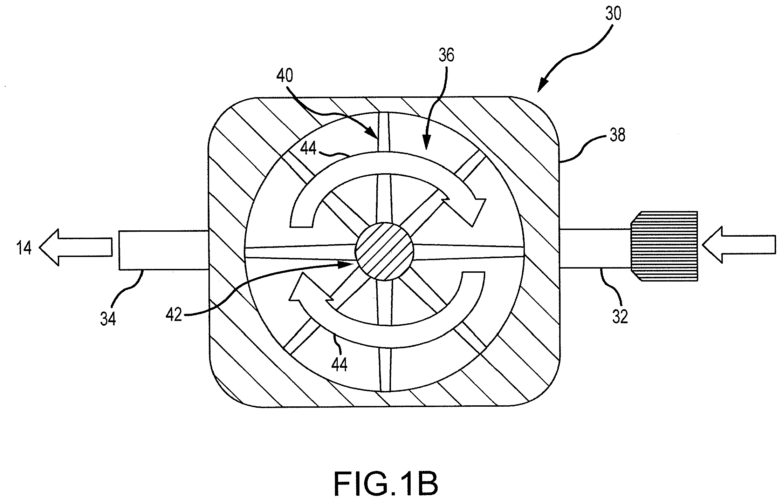

Some embodiments of control and modulation devices disclosed herein may send and/or receive a sensor signal so as to coordinate a valving, controlling, or otherwise modulating function on an injection agent before the agent enters an intended target injection site. Modulation may include, for example, valving (or otherwise modulating) an injection dispensed from an injection device. As described in U.S. Patent Application Ser. No. 13/839,771, indirect valving (or otherwise controlling mechanisms) may be proximally or distally positioned within, about, and/or upon the agent delivery system. An example of an indirect modulation control system 10 is depicted in FIGS. 1A-1D. In this example, a sensor 12 is deployed distally on a delivery catheter 14 (as seen in FIG. 1A) and a modulating device 30 (of FIG. 1B) is provided proximally. The sensor 12 of FIG. 1A is an exemplary pressure sensor positioned on the distal tip of the delivery catheter 14. As described previously, this is only one example of a type of sensor that may be used in obtaining a signal to synchronize the delivery of medium with the blood flow rate. Moreover, FIG. 1A illustrates the positioning of the sensor 12 upon the distal tip of the delivery catheter 14 within the aorta 16 to the left coronary artery 18, off the aortic root 20. The exemplary positioning of the sensor 12 in FIG. 1A should not be limited to that shown in order to perform the functions described herein, since there may be a multitude of sensor types (and commensurate signals) positioned at various locations on (i.e., as a function of respiration), through (i.e., as a function of imaging) and within the body (i.e., as a function of a variable proximate a target delivery site). Clearly, even the placement of a distal pressure sensor in exemplary FIG. 1A could take many forms, such as: a pressure wire alongside the catheter, a lumen within the catheter body for pressure measurement, a pressure sensor deployed within the distal tip of the catheter, and a pressure sensor deployed distally of the distal tip of the catheter and into the target vessel, to name but a few.

Referring to FIG. 1B, modulating device 30 may include an inlet port 32 (from the injection device) and an outlet port 34 (to the delivery catheter 14). The flow of injection fluid may pass through the injection port 32 and into a fluid chamber 36 within a body or housing 38 of the modulator 30. The modulator 30 may have a plurality of vane/plates 40 attached to a cylindrical hub 42 disposed within the fluid chamber 36. The vanes 40 and hub 42 may be formed to define a "pinwheel" structure of vane-hub that is capable of rotating freely (relative to fluid chamber 36 and body 38 of modulator 30) upon the injection of medium into the fluid chamber 36 through the injection port 32. The hub 42 may be designed to preferentially rotate in one direction. For example, FIG. 1B illustrates the preferential flow of fluid and rotation of the vane-hub, in a clockwise direction, via flow arrows 44. From the fluid chamber 36, injection fluid may flow out of the modulator 30 via the outlet port 34.

One advantage of the vane-hub modulator 30 depicted in FIG. 1B is that it may be easy to measure, or otherwise identify, the total volume of injection fluid delivered through the modulating device 30 (over time) since the volume of fluid passing through the device 30 during one rotation of the vane 40 or hub 42 may be easily determined, and the number of rotations simply counted by a counting mechanism. Alternatively, each "cell" of fluid between adjacent vanes 40 may be readily counted by a counting mechanism, that may include a magnetic, mechanical, ultrasonic, infrared or similar measurement device capable of identifying the number of times a vane 40 and/or some other element of the device 30 has passed within its field of measurement, or by determining the number of times the axis of the hub 42 has rotated. The output of such a counting mechanism may be utilized to determine and display (in real time) the total volume of medium used during a procedure. Advantageously, in the management of medium injected, an operator or physician may readily see the amount of medium used (as determined by the counting mechanism and presented by a suitable display or indicative output). The determination of the volume (via calculations or conversions based on, for example, counted rotations) may be performed as part of the counting device, or may be performed by a display device. In addition to providing volume measurements, the counting mechanism, signal, or display may incorporate various algorithms to alert the operator before or when maximum volume of agent has been administered (based upon an operator-determined value, Maximum Acceptable Contrast Dose, Gurm ratio, etc.). For example, the Maximum Acceptable Contrast Dose index, as described by Cigarroa, et al. (June 1989) "Dosing of Contrast Material to Prevent Nephropathy in Patients with Renal Disease" Am Jour of Med. 649-652, suggests that a maximum amount of contrast injected (in mL) be equal to 5ml x body weight (Kg)/Baseline Serum Creatinine level (in mg/dL). In another example, the maximum amount of contrast injected (in mL) as described in Gurm, et al. "Renal Function-Based Dosing to Define Safe Limits of Radiographic Contrast Media in Patients Undergoing Percutaneous Coronary Interventions" JACC 2011:58:907-14, suggests that the maximum contrast used (in mL) should be less than, or equal to, 2 if it is divided by a calculated Creatinine Clearance (mL/min) of the patient. Regardless of the indicator utilized, the system may include a display that not only provides total volume used, but also warns the operator of use as compared to one or more indicators of a maximum administration.

Continuing with the description of the exemplary modulation device 30 shown in FIGS. 1B-1C, the vane-hub modulator 30 may include two components. The first, the body 38 (described above) may be situated adjacent a controller/actuator 46 and may include the input port 32, the output port 34 and the fluid chamber 36 with rotating vane 40 and hub 42. The body 38 may come into contact with bodily fluids and, accordingly, may be disposable. The controller/actuator 46 may also include a brake mechanism 48, sensor signal, receiver 50, and the like may be used to clutch, brake, or otherwise inhibit the rotation of the hub 42 so as to provide resistance to rotation. The resistance induced to the rotation may be coordinated with a signal from sensor 12 of FIG. 1A, so as to modulate an injection from an injector to improve an agent fluid flow.

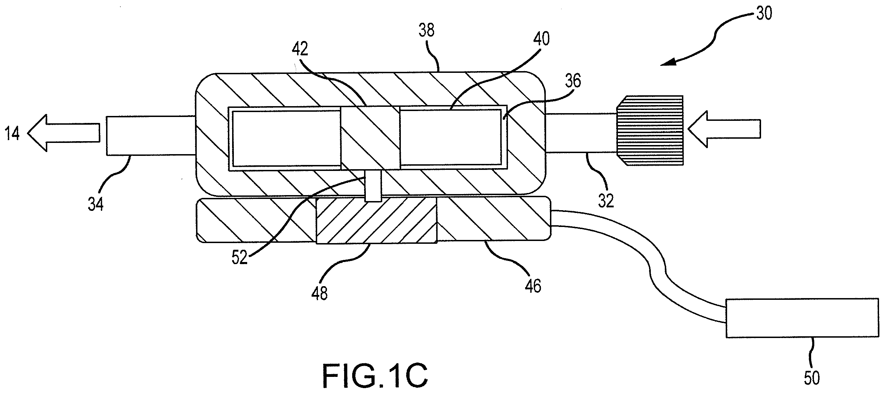

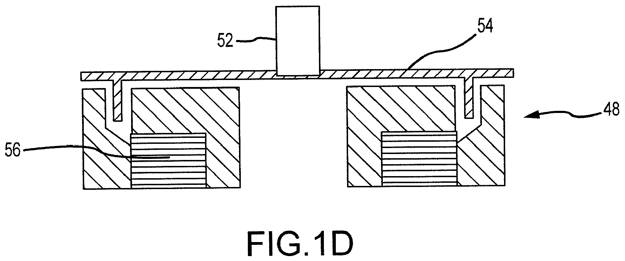

The braking, or clutching, of the modulator 30 of FIG. 1C may be performed through a variety of mechanisms, to include, for example, mechanical, hydromechanical, electromechanical, electromagnetic, chemomechanical, etc. FIG. 1C illustrates one such mechanism 48 for braking a shaft 52 of the hub 42, using electromagnetic force. The exemplary braking structure 48 is further detailed in FIG. 1D, wherein the longitudinal shaft 52 of the hub 42 is coupled to a hysteresis plate or disc 54 positioned within a magnetic coil 56. When electricity is applied to the magnetic coil 56, a magnetic flux is transferred to the hysteresis disc 54 (as it passes through the field) causing a magnetic "drag" on the disc 54. The drag, or braking, applied to the hysteresis disc 54 (and thus the shaft 52 of the hub 42) may be increased or decreased with increasing or decreasing voltage applied to the magnetic field to modulate the flow of medium as intended. When electrical current is removed, the connected disc 54 may rotate freely about an axis of shaft 52. Upon modulating, braking mechanism 48 of FIG. ID may increase the drag (reduce the flow rate) of the agent as needed to improve the flow profile of the agent or fluid.

FIGS. 1A-AD describe one system to regulate the flow profile and determine the volume of injection agent through a modulator, and as such, are intended to illustrate the modulation monitoring, control, and measurement concepts disclosed herein without limitation. Therefore, this embodiment is but one example how one might use a modulator device and a measurement device to control the delivery of an agent, as well as measure the amount of agent delivered.

Other embodiments including devices and methods in quantitative assessment, or otherwise measurement, of the volume of delivery of an agent are described below. It is to be understood that these measurement devices may also be used in combination with a variety of agent modulators and the description is intended to be exemplary and not limiting.

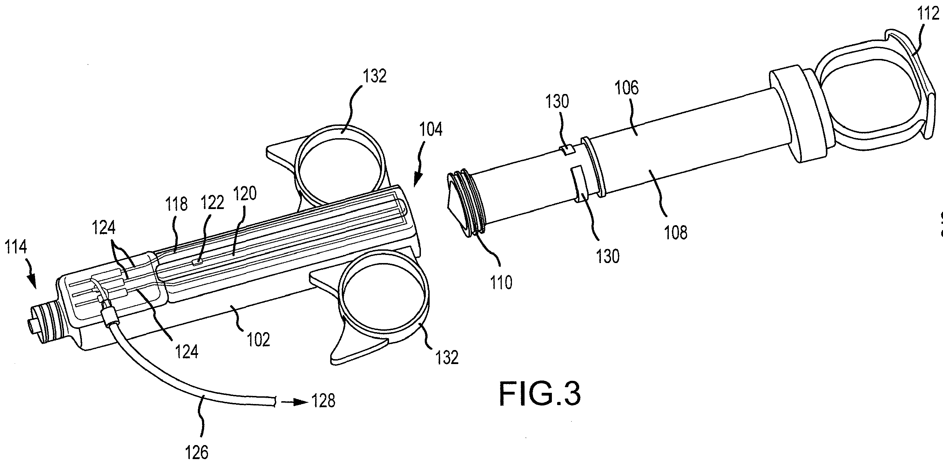

FIGS. 2 and 3 depict a perspective view and a perspective exploded view, respectively, of a monitoring syringe 100. The monitoring syringe 100 includes a syringe housing 102 (or chamber) defining an inner bore 104. A plunger 106 including a shaft 108 and a piston 110 is slidably received in the bore 104. More specifically, the piston 110 is slidably engaged with an interior surface of the bore 104 and linear movement M of the shaft 108 within the bore 104 moves the piston 110. Movement M is along the syringe axis Aₛ. The plunger 106 is moved back and forth within the bore 104 by the movement of a thumb-ring 112, as described in more detail below. As the plunger 106 is moved M in a direction towards the discharge end 114 of the syringe housing 102, the fluid contained therein is discharged into a tube or needle (not shown) and delivered to a patient. Note that throughout the description a cylindrical-type chamber 102 and inner bore 104 are described; however, it is contemplated that there might be a variety of constructions of a housing/bore 102/104 and plunger that provide the function as anticipated herein and the shape (including rectangular, ovular, triangular cross-section, etc.), in and of itself, should not be limiting.

In the depicted embodiment, a potentiometer housing 118 is secured to an exterior surface of the syringe housing 102. The potentiometer housing 118 encloses a linear potentiometer 120. In certain embodiments, the linear potentiometer 120 may be manufactured by various manufacturers. A wiper magnet 122 is also disposed within the potentiometer housing 118. One or more leads or wires 124 extend from an end of the potentiometer housing 118. The wires are joined within a cable 126 that connects at an end 128 to an interface unit that analyzes the output of the potentiometer 120 and provides this information to a user of the monitoring syringe 100, typically on a display. The displayed information may include volume of the chamber, volume remaining, volume dispensed, fluid type, flow rate, fluid pressure or temperature and/or other information, as required or desired for a particular application. In the depicted embodiment, a plurality of plunger magnets 130 are secured to the shaft 108 of the plunger 106. The plunger magnets 130 are "C" or arc-shaped and substantially surround the shaft 108 of the plunger 106. Movement of the plunger magnets 130 (due to movement of the plunger 106) moves the wiper magnet 122. The changing position of the wiper magnet 122 changes the electrical output of the linear potentiometer 120. These changes allow the interface to determine the various types of information listed above, based on a known diameter and length of the bore 104 of the syringe housing 102. Two finger rings 132 receive the fingers of a user during use.

The interaction between the magnetic fields produced by the wiper magnet 122 and the plunger magnets 130 has been discovered to produce extremely accurate movement of the wiper magnet 122. This accurate movement results in very accurate signals sent by the potentiometer 120 to the interface. In the depicted embodiment, the plunger magnet 130 is disposed within the syringe housing 102 (about the plunger 106) and the wiper magnet 122 is disposed within the potentiometer housing 118. Accordingly, these magnets are not in contact. It has been discovered, in various configurations, that utilizing a magnetic material and a non-magnetic material may produce less accurate results. As an example, during testing, a non-magnetic material was utilized as a wiper within the potentiometer housing 118. Magnets 130 such as the types described herein were secured to the plunger 106. It was noted that even though the magnetic field produced by the plunger magnets 130 were able to move the wiper along the potentiometer 120, the movement of the wiper could be susceptible to lagging behind the movement of the plunger magnet 130. In this configuration, if the plunger 106 was actuated at too high of a rate, friction within the potentiometer housing 118 might cause the wiper to drag to such an extent that the wiper was released from the magnetic field of the plunger magnets 130, and thus making inaccurate measurements. When magnetic material was used for both the wiper magnet 122 and the plunger magnets 130, very little lag occurred as the opposing magnetic fields were better able to sustain matching movement of the wiper magnet 122 and the plunger magnet 130.

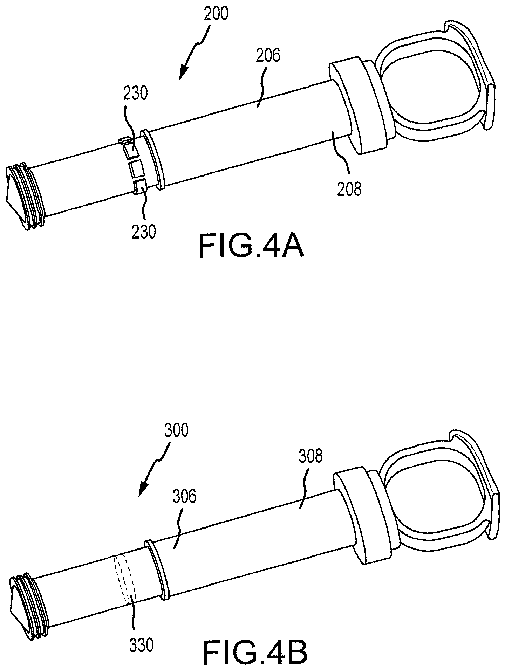

FIG. 4A depicts a partial enlarged perspective view of another embodiment of a monitoring syringe 200. In this embodiment, a plunger 206 includes a shaft 208. Rather than the two arc magnets depicted above in FIGS. 2 and 3, the depicted embodiment includes a plurality of substantially square plunger magnets 230 that are secured directly to the plunger shaft 208. The shape of the plunger 206 (or shaft 208) and the magnets 230 may not be critical. It is notable, however, that the magnets 230 are disposed about the outer circumference of the shaft 208. And, some portion of the plunger 206 (portion 110 as shown in Figure 2, for example) should be in sealing, but slideable, relationship within chamber 104. FIG. 4B depicts a partial enlarged perspective view of another embodiment of a monitoring syringe 300. Here, a plunger magnet 330 is disposed within a shaft 308 of a plunger 306. The magnet 330 may be secured in place with an adhesive or may be press-fit to a hollow portion of the shaft 308. Further, it is to be understood that there may be a variety of ways to construct the plunger/shaft 206/208 to accomplish the same function. As an example, magnet sections 330 might be a separate element affixing both ends of the shaft 208 together.

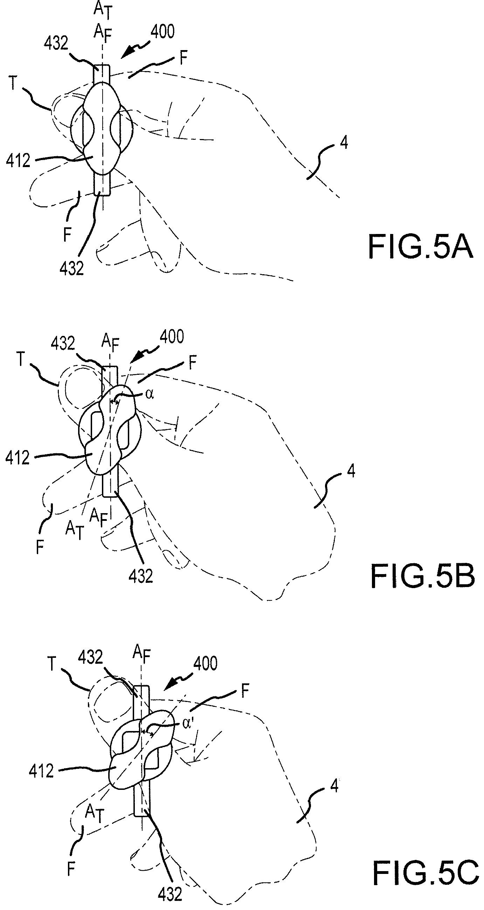

It should be noted that other orientations and positions of the plunger magnet or magnets are contemplated. In certain embodiments, the plunger magnets are oriented so as to substantially surround the shaft 108 of the plunger 106. In other embodiments, the magnet, for example, may be located within the shaft of the plunger. Regardless, the plunger magnet or magnets are oriented such that the plunger magnet remains within the magnetic field of the wiper magnet and vice versa, even during axial rotation of the plunger (that is about the syringe axis Aₛ of FIG. 2). Without this relationship, one might receive inaccurate readings from the monitoring syringe device. If a single discrete plunger magnet is utilized, and disposed only on a small portion of the plunger, the chances of the wiper magnet passing outside of the plunger magnet magnetic field can greatly increase, especially as the plunger is rotated during use. FIGS. 5A-5C illustrate this problem.

In FIG. 5A, a user U holds a monitoring syringe 400 in their right hand, with the plunger in the forward-most position. The user's thumb T is located within the thumb ring 412 and first two fingers F are located within the finger rings 432. In the depicted embodiment, the syringe housing defines the syringe axis A_{S} (depicted in FIG. 2). Other axes relevant to the present discussion are also depicted. First, a finger ring axis A_{F} bisects the two finger rings 432 and is substantially orthogonal to the syringe axis A_{S}. Second, a thumb ring axis A_{T}, bisects a thumb ring 412, which is used to withdraw and advance the plunger. The thumb ring axis A_{T} is also substantially orthogonal to the syringe axis A. It should be noted that the position of the finger rings 432, in this example, is fixed on the syringe housing, much like the position of the potentiometer may be fixed on the chamber housing. Accordingly, the changing angular position of the thumb ring axis A_{T} relative to the finger ring axis A_{F} could also be described as the changing position of the thumb ring axis A_{T} to any element on the syringe housing.

When in the initial position of FIG. 5A, the thumb ring axis A_{T} may be substantially parallel to the finger ring axis A_{F}. As the user's thumb T is pulled back, as depicted in FIG. 5B, the physiology of the thumb T may cause a rotation of the plunger (as indicated by the position of the thumb ring axis A_{T}). Here, an angle α (when viewed from the end of the syringe 400, as depicted) may open between the finger ring axis A_{F} and the thumb ring axis A_{T}. A more pronounced angle α' may be produced as the thumb T is withdrawn to the final position, as depicted in FIG. 5C. Thus, orientation of the magnets about the shaft of the plunger may help ensure that the magnetic fields produced by the plunger magnet and wiper magnet remain aligned, even as the angular position of the plunger within the syringe housing changes during use. If only a single discrete plunger magnet was disposed on the outside surface of the plunger, it might be moved out of the magnetic field of the wiper magnet as the plunger rotated about the syringe axis A_{S}, as depicted by the increasing angles α, α' between the thumb ring axis A_{T} and finger ring axis A_{F}. Similar angular movement of the thumb ring axis A_{T} is likely as the plunger is returned to the original position. Of course, a user U could carefully keep the angular position of the plunger fixed within the syringe/chamber housing during use. Or one might design a device incapable of rotation. However, both options might result in lower acceptance by users, practically and/or physiologically. Another advantage to the depicted device is that it may be used by both the left and the right hands of a user, without requiring any additional configuration or modifications. Accordingly, the technologies disclosed herein include one or more plunger magnets configured to maintain the magnetic field about the wiper magnet, thus ensuring that normal rotation of the plunger during use does not adversely affect operation of device, or readings from the potentiometer.

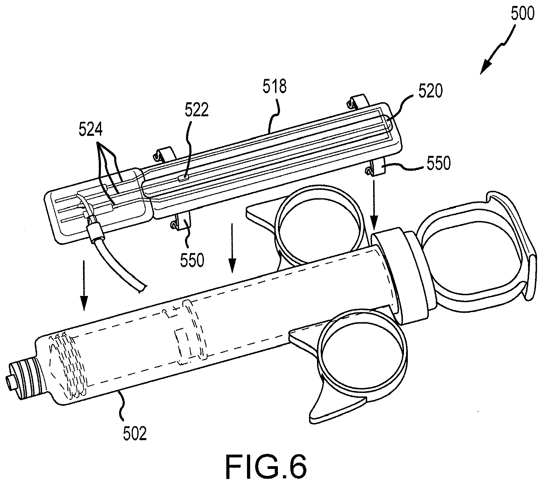

FIG. 6 depicts another embodiment of a monitoring syringe 500. In this case, the potentiometer housing 518, containing the potentiometer 520, wiper magnet 522, and wires 524, is detachable secured to the syringe housing 502. The potentiometer housing 520 may be secured with clips, C-clamps, resilient catches, or other elements (such as 550) that allow the potentiometer housing 520 to be removed from the syringe housing 502. Such a configuration may be desirable so the potentiometer housing 518 and related components may be reused on a different syringe, typically after a medical procedure. The potentiometer housing 518 may be removed from a first syringe housing 502 and reattached to a second syringe housing at a later time. Once the wires 524 are reconnected to the interface (as described above) a calibration program may be executed so as to calibrate the potentiometer 520 for the new syringe.

The monitoring systems described herein may be utilized to deliver any types of fluids to a patient during a medical procedure. Such fluids may include medium (media), agents, substances, materials, medicaments, and the like. It should be noted that these terms are used generically herein to describe a variety of fluidal materials that may include, at least in part, a substance used in the performance of a diagnostic, therapeutic or/and prophylactic medical procedure and such use is not intended to be limiting. It should be understood that the medium delivery modulation and/or measurement devices and methods described herein are not limited to the particular, representative embodiments as described, since variations may be made to these embodiments without departing from the scope and spirit of the disclosure. Likewise, terminology employed in the description of embodiments is not intended to be limiting and is used merely for the purpose of conveyance of the concept. Unless defined otherwise, all technical and scientific terms used herein have the same meaning as commonly understood to one of ordinary skill in the art of which the disclosed devices and methods pertain.

While there have been described herein what are to be considered exemplary and preferred embodiments of the present technology, other modifications of the technology will become apparent to those skilled in the art from the teachings herein. The particular methods of manufacture and geometries disclosed herein are exemplary in nature and are not to be considered limiting.

## Claims

1. An apparatus comprising:
a syringe housing (102, 502);
a plunger (106, 206, 306) slidably received within the syringe housing (102, 502); such plunger comprising a shaft (108);
a potentiometer housing (118, 518);
a potentiometer (120, 520) disposed within the potentiometer housing (118, 518); **characterized in that**
the apparatus comprises at least one plunger magnet (130, 230, 330) secured to the shaft (108, 208, 308) of the plunger (106, 206, 306);
said potentiometer housing (118, 518) is fixed to the syringe housing;
and **in that**
a wiper magnet (122, 522) is slidably received within the potentiometer housing, wherein a movement of the at least one plunger magnet (130, 230, 330) causes a corresponding movement of the wiper magnet (122, 522); and, **in that**
such at least one plunger magnet (130, 230, 330) is oriented such that said at least one plunger magnet remains within the magnetic field of the wiper magnet and vice versa, even during axial rotation of the plunger.

2. The apparatus of claim 1, further comprising a plurality of leads (124) extending from the potentiometer (120, 520).

3. The apparatus of claim 2, further comprising an interface for connecting the plurality of leads (124) to a measuring device, and wherein the measuring device displays a total volume injected and emits a warning of a critical outcome.

4. The apparatus of claim 1, wherein the potentiometer housing (118, 518) is releasably fixed to the syringe housing (102, 502).

5. The apparatus of claim 4, further comprising means for releasably securing the potentiometer housing (118, 518) to the syringe housing (102, 502).

6. The apparatus of claim 1, wherein,
the plunger (106, 206, 306) is also rotatably received within the syringe housing (102, 502); and,
wherein the wiper magnet is adapted to move linearly along the potentiometer (120, 520) in tandem with rotational and linear movement of the plunger (106, 206, 306).

7. The apparatus of claim 6, further comprising a lead (124) for sending an output signal from the potentiometer (120, 520) to an interface, wherein the interface displays a total volume injected and emits a warning of a critical outcome.

8. The apparatus of claim 7, wherein the output signal varies based on a position of the wiper magnet relative to the potentiometer (120, 520).

9. The apparatus of claim 6, wherein the wiper magnet is disposed proximate the potentiometer (120, 520).

10. The apparatus of claim 9, wherein the at least one plunger magnet (130, 230, 330) is fixed to the plunger (106, 206, 306).

11. The apparatus of claim 1, wherein the at least one plunger magnet (130, 230, 330) comprises a plurality of plunger magnets secured to and disposed so as to substantially surround the shaft (108) of the plunger (106, 206, 306).

12. The apparatus of claim 1, wherein at least one plunger magnet (130, 230, 330) aligned with the wiper magnet.

## Patentansprüche

1. Vorrichtung, umfassend:
ein Spritzengehäuse (102, 502);
einen Kolben (106, 206, 306), der gleitend in dem Spritzengehäuse (102, 502) aufgenommen ist; wobei ein solcher Kolben einen Schaft (108) umfasst;
ein Potentiometergehäuse (118, 518);
ein Potentiometer (120, 520), das innerhalb des Potentiometergehäuses (118, 518) angeordnet ist;
**dadurch gekennzeichnet, dass**
die Vorrichtung zumindest einen Kolbenmagneten (130, 230, 330) umfasst, der am Schaft (108, 208, 308) des Kolbens (106, 206, 306) angeordnet ist;
wobei das Potentiometergehäuse (118, 518) am Spritzengehäuse gesichert ist;
und dass
ein Schleifmagnet (122, 522) gleitend innerhalb des Potentiometergehäuses aufgenommen ist, wobei eine Bewegung des zumindest einen Kolbenmagneten (130, 230, 330) eine entsprechende Bewegung des Schleifmagneten (122, 522) bewirkt; und dadurch, dass
der zumindest eine Kolbenmagnet (130, 230, 330) so ausgerichtet ist, dass der zumindest eine Kolbenmagnet innerhalb des Magnetfeldes des Schleifmagneten bleibt und umgekehrt, auch während einer axialen Drehung des Kolbens.

2. Vorrichtung nach Anspruch 1, weiterhin umfassend eine Vielzahl von Leitungen (124), die vom Potentiometer (120, 520) ausgehen.

3. Vorrichtung nach Anspruch 2, die ferner eine Schnittstelle zum Verbinden der Vielzahl von Leitungen (124) mit einer Messvorrichtung umfasst, und wobei die Messvorrichtung ein injiziertes Gesamtvolumen anzeigt und eine Warnung vor einem kritischen Ergebnis ausgibt.

4. Vorrichtung nach Anspruch 1, wobei das Potentiometergehäuse (118, 518) lösbar am Spritzengehäuse (102, 502) angeordnet ist.

5. Vorrichtung nach Anspruch 4, die ferner Mittel zur lösbaren Befestigung des Potentiometergehäuses (118, 518) am Spritzengehäuse (102, 502) umfasst.

6. Vorrichtung nach Anspruch 1, bei der der Kolben (106, 206, 306) ebenfalls drehbar im Spritzengehäuse (102, 502) aufgenommen ist; und,
wobei der Schleifmagnet so angepasst ist, dass er sich linear entlang des Potentiometers (120, 520) zusammen mit der Dreh- und Linearbewegung des Kolbens (106, 206, 306) bewegt.

7. Vorrichtung nach Anspruch 6, die ferner eine Leitung (124) zum Senden eines Ausgangssignals von dem Potentiometer (120, 520) an eine Schnittstelle umfasst, wobei die Schnittstelle ein injiziertes Gesamtvolumen anzeigt und eine Warnung vor einem kritischen Ergebnis ausgibt.

8. Vorrichtung nach Anspruch 7, wobei das Ausgangssignal auf der Grundlage einer Position des Schleifmagneten relativ zu dem Potentiometer (120, 520) variiert.

9. Vorrichtung nach Anspruch 6, bei der der Schleifmagnet in der Nähe des Potentiometers (120, 520) angeordnet ist.

10. Vorrichtung nach Anspruch 9, wobei der zumindest eine Kolbenmagnet (130, 230, 330) an dem Kolben (106, 206, 306) befestigt ist.

11. Vorrichtung nach Anspruch 1, wobei der zumindest eine Kolbenmagnet (130, 230, 330) eine Vielzahl von Kolbenmagneten umfasst, die an dem Kolben (106, 206, 306) befestigt und so angeordnet sind, dass sie den Schaft (108) des Kolbens (106, 206, 306) im Wesentlichen umgeben.

12. Vorrichtung nach Anspruch 1, bei der zumindest ein Kolbenmagnet (130, 230, 330) mit dem Schleifmagneten ausgerichtet ist.

## Revendications

1. Appareil comprenant :
un boîtier de seringue (102, 502) ;
un piston (106, 206, 306) reçu de manière coulissante au sein du boîtier de seringue (102, 502) ; un tel piston comprenant une tige (108) ;
un boîtier de potentiomètre (118, 518) ;
un potentiomètre (120, 520) disposé au sein du boîtier de potentiomètre (118, 518) ;
**caractérisé en ce que**
l'appareil comprend au moins un aimant de piston (130, 230, 330) rattaché à la tige (108, 208, 308) du piston (106, 206, 306) ;
ledit boîtier de potentiomètre (118, 518) est fixé au boitier de seringue ;
et **en ce que**
un aimant de racleur (122, 522) est reçu de manière coulissante au sein du boîtier de potentiomètre, dans lequel un mouvement de l'au moins un aimant de piston (130, 230, 330) provoque un mouvement correspondant de l'aimant de racleur (122, 522) ; et, **en ce que** un tel au moins un aimant de piston (130, 230, 330) est orienté de sorte que ledit au moins un aimant de piston reste au sein du champ magnétique de l'aimant de racleur et inversement, même pendant une rotation axiale du piston.

2. Appareil selon la revendication 1, comprenant en outre une pluralité de connecteurs (124) s'étendant à partir du potentiomètre (120, 250).

3. Appareil selon la revendication 2, comprenant en outre une interface pour connecter la pluralité de connecteurs (124) à un dispositif de mesure, et dans lequel le dispositif de mesure affiche un volume total injecté et émet un avertissement concernant une issue critique.

4. Appareil selon la revendication 1, dans lequel le boîtier de potentiomètre (118, 518) est fixé de manière amovible au boîtier de seringue (102, 502).

5. Appareil selon la revendication 4, comprenant en outre des moyens pour rattacher de manière amovible le boîtier de potentiomètre (118, 518) au boîtier de seringue (102, 502) .

6. Appareil selon la revendication 1, dans lequel le piston (106, 206, 306) est également reçu de manière rotative au sein du boîtier de seringue (102, 502) ; et dans lequel l'aimant de racleur est adapté pour se déplacer de manière linéaire le long du potentiomètre (120, 520) en tandem avec un mouvement rotatif et linéaire du piston (106, 206, 306).

7. Appareil selon la revendication 6, comprenant en outre un fil (124) pour envoyer un signal de sortie du potentiomètre (120, 520) à une interface, dans lequel l'interface affiche un volume total injecté et émet un avertissement concernant une issue critique.

8. Appareil selon la revendication 7, dans lequel le signal de sortie varie sur la base d'une position de l'aimant de racleur par rapport au potentiomètre (120, 520) .

9. Appareil selon la revendication 6, dans lequel l'aimant de racleur est disposé à proximité du potentiomètre (120, 520).

10. Appareil selon la revendication 9, dans lequel l'au moins un aimant de piston (130, 230, 330) est fixé au piston (106, 206, 306).

11. Appareil selon la revendication 1, dans lequel l'au moins un aimant de piston (130, 230, 330) comprend une pluralité d'aimants de piston rattachés à la tige (108) du piston (106, 206, 306) et disposés de façon à l'entourer sensiblement.

12. Appareil selon la revendication 1, dans lequel au moins un aimant de piston (130, 230, 330) est aligné avec l'aimant de racleur.
